# FASCICULE DE BREVET EUROPEEN

(11) **EP 2 736 324 B1**
(45) Date de publication et mention de la délivrance du brevet: **15.02.2017**
(21) Numéro de dépôt: 12735160.9
(22) Date de dépôt: 17.07.2012
(51) Int. Cl.: A61B 10/02, A01K 11/00, A61B 10/00

(54) **SYSTÈME DE PRÉLÈVEMENT D'AU MOINS UN ÉCHANTILLON DE TISSU ANIMAL, DISPOSITIF DE PRÉLÈVEMENT, DISPOSITIF DE STOCKAGE, ET PROCÉDÉ DE FABRICATION CORRESPONDANTS**
SYSTEM ZUR ENTNAHME VON MINDESTENS EINER PROBE AUS TIERGEWEBE UND ENTSPRECHENDE PROBENNAHMEVORRICHTUNG, AUFBEWAHRUNGSVORRICHTUNG UND HERSTELLUNGSVERFAHREN
SYSTEM FOR COLLECTING AT LEAST ONE SAMPLE OF ANIMAL TISSUE, AND CORRESPONDING SAMPLING DEVICE, STORAGE DEVICE, AND MANUFACTURING METHOD

(30) Priorité: 28.07.2011 FR 1156897
(43) Date de publication de la demande: 04.06.2014
(73) Titulaire: Allflex Europe, 35500 Vitré (FR)
(72) Inventeur: DECALUWE, Johan, F-53000 Laval (FR); TEYCHENE, Bruno, F-81430 Mouzieys-Teulet (FR); HILPERT, Jean-Jacques, F-35500 Vitré (FR); DESTOUMIEUX, Jean-Jacques, F-81380 Lescure D'albigeois (FR)
(74) Mandataire: Vidon Brevets & Stratégie
(86) Numéro de dépôt international: PCT/EP2012/064018
(87) Numéro de publication internationale: WO 2013/014034

(56) Documents cités:
- WO-A1-2008/152980
- WO-A1-2009/076469
- WO-A1-2011/047902
- WO-A2-2006/000869

## Description

### 1. Domaine de l'invention

Le domaine de l'invention est celui du contrôle et/ou de l'identification des animaux.

Plus précisément, l'invention concerne le prélèvement et le stockage de tissu animal, permettant notamment de conserver des cellules portant des caractéristiques biologiques ou biochimiques de l'animal, par exemple pour identifier ultérieurement l'animal ou détecter des maladies de l'animal. On note qu'un tel prélèvement peut être effectué sur toute espèce animale (bovins, ovins, porcins, caprins, volatiles, poissons, etc), avec ou sans pose simultanée d'une marque d'identification.

Encore plus précisément, l'invention concerne les moyens de stockage et de conservation d'un tel prélèvement.

### 2. Art antérieur

Afin d'améliorer le suivi du cheptel, améliorer la productivité (en éliminant les animaux malades, ou en recherchant des caractéristiques génétiques singulières par exemple), et/ou garantir l'origine des animaux destinés notamment à la consommation (par exemple en détectant des maladies), il est de plus en plus souvent procédé à un ou plusieurs prélèvements de tissu des animaux concernés.

Un tel prélèvement peut être effectué sur l'animal lors de la pose d'une marque d'identification de l'animal, à la naissance par exemple, ou ultérieurement, tout au long de l'existence de l'animal. Plusieurs prélèvements peuvent ainsi être effectués, notamment pour détecter des maladies ou certifier l'identité de l'animal, par exemple par comparaison des séquences ADN.

Les techniques de prélèvement de tissu classiquement mises en oeuvre pour prélever un échantillon de tissu indépendamment de la pose d'une marque d'identification reposent sur l'utilisation d'un emporte-pièce formant ou comprenant un élément de coupe, présentant une arête coupante de forme généralement circulaire (à contact continu ou en dents-de-scie), destiné à découper un échantillon de tissu animal et à le collecter dans un logement.

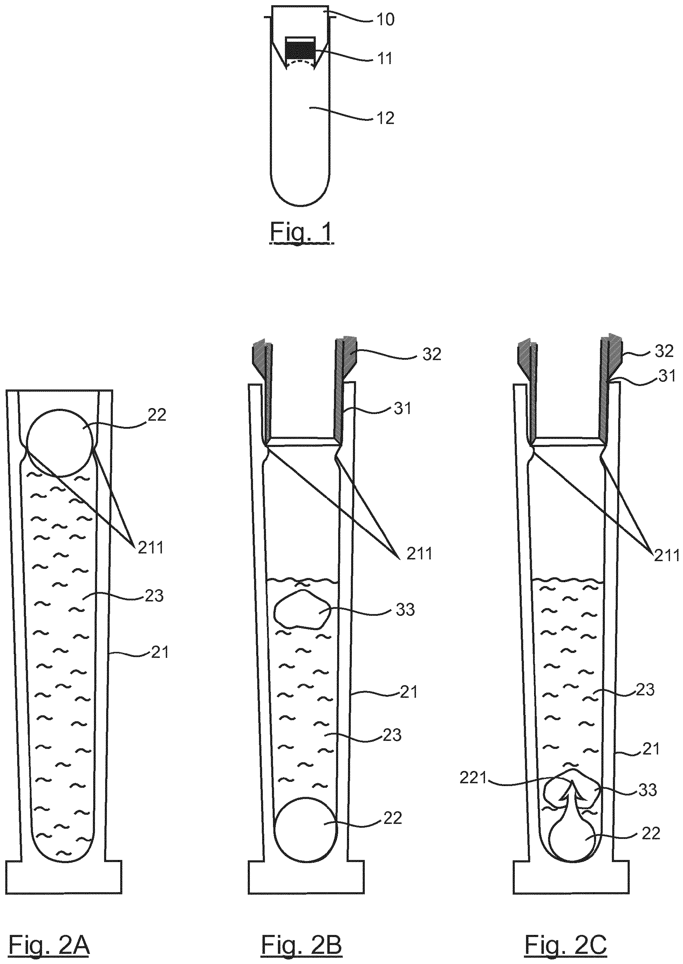

Comme illustré en figure 1, l'emporte-pièce 10 est classiquement fixé à une première mâchoire d'un outil de prélèvement, une pince par exemple. Un microtube 12 destiné à stocker l'échantillon après prélèvement est fixé à une deuxième mâchoire de l'outil. Un tel microtube 12 est classiquement fermé par un opercule avant prélèvement, notamment pour protéger l'intérieur du microtube 12 de l'environnement extérieur.

Lors de l'actionnement de l'outil, l'élément de coupe 10 vient découper la peau de l'animal, déchirer l'opercule servant de couvercle au microtube 12, et s'insérer au moins partiellement dans le microtube 12. L'emporte-pièce 10 présente donc classiquement un diamètre légèrement inférieur à celui du microtube 12, de façon à servir de bouchon au microtube.

Une fois collecté, l'échantillon de tissu animal peut être stocké et/ou transmis à un laboratoire pour analyse.

Grâce à cette technique de prélèvement, l'échantillon 11 est directement inséré dans le microtube 12 après perforation de l'opercule, ce qui limite le risque de contamination de l'échantillon.

Néanmoins, un inconvénient de cette technique est que l'échantillon prélevé reste généralement coincé dans le logement prévu à cet effet dans l'élément de coupe après prélèvement.

L'emporte-pièce doit alors être retiré ou découpé avec précaution lors d'une analyse ultérieure de l'échantillon, afin d'éviter de perdre ou contaminer l'échantillon.

L'opération d'extraction de l'échantillon de tissu collecté dans le logement se révèle donc malaisée et/ou complexe, et présente un risque de perte de l'échantillon, ainsi qu'un risque de coupure pour l'utilisateur.

De plus, la conservation de l'échantillon dans l'élément de coupe n'est pas optimale.

Il existe donc un besoin pour une nouvelle technique de prélèvement/stockage de tissus animal ne présentant pas ces inconvénients de l'art.

D'autres techniques de prélèvement de tissu sont divulguées dans les documents WO 2006/000869-A2, WO 2009/076469-A1, WO 2008/152980-A1 et WO 2011/047902-A1.

### 3. Exposé de l'invention

L'invention propose une solution nouvelle qui ne présente pas l'ensemble de ces inconvénients de l'art antérieur, sous la forme d'un système de prélèvement d'au moins un échantillon de tissu animal, comprenant :
- un dispositif de prélèvement comprenant au moins un élément de coupe destiné à découper un échantillon de tissu animal, et
- un dispositif de stockage comprenant un tube de réception destiné à recevoir l'échantillon.

Selon l'invention, un tel système comprend également un élément lesté configuré pour prendre au moins deux positions, dont une première position antérieure au prélèvement de l'échantillon, dans laquelle l'élément lesté est maintenu dans le dispositif de prélèvement ou dans le dispositif de stockage, et une deuxième position postérieure au prélèvement de l'échantillon, dans laquelle l'élément lesté est libre dans le tube, l'élément lesté étant poussé à l'intérieur du tube lors du prélèvement.

L 'élément lesté est une sphère ou un cylindre présentant une densité supérieure ou égale à celle d'au moins un agent spécifique contenu dans le tube.

Ainsi, l'invention repose sur l'utilisation d'un nouvel élément original dans les systèmes de prélèvement, fixé dans le dispositif de prélèvement ou dans le dispositif de stockage dans une première position, et libre dans le dispositif de stockage dans une deuxième position, pouvant remplir plusieurs fonctions selon la position qu'il occupe.

Par exemple, dans la première position, l'élément lesté est maintenu dans la partie supérieure du dispositif de stockage et obture un orifice d'entrée du tube. Dans cette première position préalable au prélèvement, correspondant par exemple à la forme sous laquelle les tubes de réception sont commercialisés, l'élément lesté remplit la fonction de bouchon du tube de réception, permettant d'éviter l'introduction d'impuretés dans le tube, et donc de contaminer l'intérieur du tube par l'environnement extérieur.

Dans le cas où le tube contient au moins un agent spécifique du type agent conservateur, agent dessiccant, agent réactif, agent de préparation de l'échantillon, etc, l'élément lesté formant bouchon évite la perte de l'agent spécifique (qui peut notamment se présenter sous la forme d'un gel, une crème, une graisse, un liquide, une poudre, un gaz, une mousse imprégnée, etc) en assurant l'étanchéité du tube avant son utilisation.

En particulier, un tel agent permet d'améliorer la conservation de l'échantillon, de le préparer en vue d'un traitement ultérieur comme une analyse ADN et/ou de le traiter directement. En d'autres termes, l'agent spécifique peut prendre la forme de tout produit pouvant agir sur un échantillon de tissu prélevé de l'animal.

Selon un autre exemple, dans la première position, l'élément lesté est maintenu dans le dispositif de prélèvement, à l'intérieur de l'élément de coupe.

Dans la deuxième position postérieure au prélèvement, l'élément lesté est libéré dans le tube d'échantillon.

Lorsque le tube contient au moins un agent spécifique (inséré dans le tube préalablement au prélèvement, par exemple lors de la fabrication du tube, ou inséré dans le tube postérieurement au prélèvement, par exemple lors de l'analyse de l'échantillon au niveau du laboratoire), l'élément lesté se trouve en contact avec l'agent spécifique. Il peut alors remplir la fonction d'agitateur ou mélangeur, permettant notamment de répartir l'agent spécifique sur l'ensemble de l'échantillon, par exemple en mélangeant et en homogénéisant le liquide si l'agent spécifique est présent sous forme liquide. Il peut également servir de lest, permettant de faire couler l'échantillon au fond du tube, ou encore de « pilon », permettant d'écraser ou d'effriter l'échantillon afin de faciliter les analyses ultérieures.

L' élément lesté prend la forme d'une sphère (bille) ou d'un cylindre.

Il présente de cette façon une section circulaire, adaptée à l'orifice d'entrée des tubes. De plus, il peut être inséré dans l'orifice d'entrée d'un tube quelle que soit son orientation.

En particulier, l'élément lesté présente un diamètre sensiblement égal au diamètre de l'orifice d'entrée du tube ou, dans le cas où le tube est muni d'une tête de tube, sensiblement égal au diamètre de l'ouverture centrale de la tête de tube, et supérieur au diamètre d'un orifice d'entrée d'une pipette, destinée à extraire l'échantillon du tube ou une certaine quantité d'agent spécifique.

Ainsi, l'élément lesté permet de fermer hermétiquement ou quasi-hermétiquement le tube avant utilisation (ie dans sa première position, lorsqu'il est prévu dans le dispositif de stockage). De plus, l'élément lesté n'obture pas les pipettes utilisées lors du pipetage, sa taille étant adaptée pour éviter toute aspiration dans la pipette. Par ailleurs, la densité de l'élément lesté lui permet de couler au fond du tube dans sa deuxième position. Il ne vient donc pas obstruer la pipette par contact.

L' élément lesté présente une densité supérieure ou égale à celle de l'agent spécifique.

Par exemple, il présente une densité supérieure à celle du ou des liquides utilisés pour la préservation des tissus prélevés et/ou à celle du ou des liquides utilisés lors des analyses ultérieures.

De cette façon, dans la deuxième position, l'élément lesté « tombe » au fond du tube, en entraînant éventuellement l'échantillon.

Ainsi, l'élément lesté peut notamment faciliter le mélange des réactifs dans le tube.

Selon une caractéristique particulière de l'invention, l'élément lesté présente une couleur spécifique.

Une telle couleur est préférentiellement choisie vive ou « tape-à l'oeil », comme le vert pomme. L'élément lesté, dans sa deuxième position, peut alors servir d'indicateur visuel du bon prélèvement. En effet, une fois le prélèvement effectué, l'élément lesté « tombe » au fond du tube, en entraînant éventuellement l'échantillon, et on peut visuellement contrôler la présence de l'élément lesté, de couleur vive, dans le tube.

Eventuellement, la couleur de l'élément lesté peut être modifiée au contact de l'agent spécifique et/ou de l'élément de coupe.

Selon une variante de l'invention, l'élément lesté comprend des moyens d'accroche de l'échantillon, permettant de solidariser l'échantillon à l'élément lesté.

Par exemple, de tels moyens d'accroche comprennent un élément appartenant au groupe comprenant :
- une pointe ;
- une aiguille ;
- un crochet ;
- un harpon.

Ainsi, lors du prélèvement de tissus, l'échantillon est poussé contre l'élément lesté et vient s'accrocher à l'élément lesté. Dans sa deuxième position, l'élément lesté entraîne l'échantillon dans le tube, avantageusement jusqu'au fond du tube.

On s'assure de cette façon que l'échantillon prélevé est en contact avec l'agent spécifique, ce qui permet une meilleure préservation de l'échantillon de tissu. De plus, cette variante permet de limiter la quantité d'agent nécessaire à la réaction, puisqu'une faible quantité d'agent est suffisante pour recouvrir l'échantillon s'il a « coulé » dans le fond du tube.

Selon un autre aspect de l'invention, l'élément lesté est inerte.

On entend ici par « inerte » ou « neutre » un matériau ne se décomposant pas, et ne produisant aucune réaction physique ou chimique. En d'autres termes, l'élément lesté ne se détériore pas au contact d'autres matières comme les tissus animal ou l'agent spécifique. De plus, il est compatible chimiquement avec les agents spécifiques utilisés.

Dans un autre mode de réalisation, l'élément lesté peut ne pas être inerte, et pouvoir réagir avec l'échantillon et/ou un agent spécifique prévu dans le tube (introduit avant, pendant, ou après le prélèvement).

En variante, l'élément lesté peut avoir un « noyau » inerte, et être recouvert d'un réactif, ou au contraire un noyau non inerte, pouvant contenir un tel réactif. En d'autres termes, l'élément lesté peut être composé « partiellement » d'un matériau inerte.

Selon encore un autre aspect, l'élément lesté comprend un aimant.

De cette façon, l'élément lesté comprend un matériau magnétique, et peut entrer en mouvement lorsqu'il est soumis à un champ magnétique, une plaque chauffante, etc. Il peut alors mélanger le contenu du tube pour favoriser les réactions entre l'agent spécifique et l'échantillon de tissu.

Bien entendu, d'autres matériaux pourraient être utilisés pour l'élément lesté, comme le verre, l'acier par exemple.

Dans un mode de réalisation particulier, le dispositif de stockage comprend une tête de tube comprenant un capuchon percé d'une ouverture centrale et une collerette, pouvant prendre appui sur le rebord du tube. Dans la première position, l'élément lesté peut alors obturer l'ouverture centrale.

L'utilisation d'une tête de tube procure de nombreux avantages. Tout d'abord, elle peut procurer un support sur lequel un élément de coupe peut s'appuyer pour découper correctement les tissus de l'animal. Elle permet également la fermeture du tube, par exemple par emboîtement ou clippage, dans la tête de tube, de l'élément de coupe ou d'un élément poussoir tels que définis dans la demande de brevet WO2010/066475 déposée le 31 juillet 2009 au nom du même Demandeur. De plus, la présence d'une telle tête de tube permet l'automatisation de l'ouverture des tubes par les laboratoires d'analyse, en décapsulant la tête de tube de façon que seuls l'échantillon et l'élément lesté restent à l'intérieur du tube.

Selon encore une autre caractéristique, le dispositif de prélèvement comprend un élément poussoir mobile par rapport à l'élément de coupe, permettant de pousser l'échantillon dans le dispositif de stockage après découpe de l'échantillon par l'élément de coupe, l'élément lesté étant poussé par l'élément poussoir à l'intérieur du tube lors du prélèvement.

En particulier, selon la technique actuelle, telle que décrite dans la demande de brevet WO2010/066475 précitée, l'élément poussoir pousse l'échantillon, déchire un opercule fermant le tube, et ferme le tube. Selon ce mode de réalisation de l'invention, si l'élément lesté est maintenu dans le dispositif de stockage dans sa première position, l'élément poussoir pousse l'échantillon dans l'orifice d'entrée du tube ou l'ouverture centrale de la tête de tube, qui pousse à son tour l'élément lesté dans le tube, puis l'élément poussoir ferme le tube. Si l'élément lesté est maintenu dans le dispositif de prélèvement dans sa première position, l'élément poussoir pousse l'élément lesté, qui pousse à son tour l'échantillon dans l'orifice d'entrée du tube ou l'ouverture centrale de la tête de tube, puis l'élément poussoir ferme le tube.

Un tel système de prélèvement présente ainsi une étanchéité améliorée par rapport aux techniques de l'art antérieur, et son industrialisation et le traitement en laboratoire du tube obtenu après prélèvement sont optimisés.

Dans un autre mode de réalisation, l'invention concerne un dispositif de prélèvement d'un système de prélèvement tel que décrit précédemment, comprenant un élément lesté.

Plus précisément, un tel dispositif de prélèvement d'au moins un échantillon de tissu animal comprend au moins un élément de coupe destiné à découper un échantillon de tissu animal, et est apte à coopérer avec un dispositif de stockage comprenant un tube de réception destiné à recevoir l'échantillon.

Selon l'invention, un tel dispositif de prélèvement comprend un élément lesté dans une première position antérieure au prélèvement de l'échantillon, dans laquelle l'élément lesté est maintenu dans le dispositif de prélèvement, l'élément lesté étant une sphère ou un cylindre présentant une densité supérieure ou égale à celle d'au moins un agent spécifique contenu dans le tube. L'élément lesté est configuré pour prendre au moins une deuxième position postérieure au prélèvement de l'échantillon, dans laquelle l'élément lesté est libre dans le tube de réception, l'élément lesté étant poussé à l'intérieur du tube lors du prélèvement.

Dans encore un autre mode de réalisation, l'invention concerne un dispositif de stockage d'un système de prélèvement tel que décrit précédemment, comprenant un élément lesté.

Plus précisément, un tel dispositif de stockage d'au moins un échantillon de tissu animal comprend un tube de réception destiné à recevoir un échantillon de tissu animal, et est apte à coopérer avec un dispositif de prélèvement comprenant au moins un élément de coupe destiné à découper l'échantillon.

Selon l'invention, un tel dispositif de stockage comprend un élément lesté dans une première position antérieure au prélèvement de l'échantillon, dans laquelle l'élément lesté est maintenu dans le dispositif de stockage, l'élément lesté étant une sphère ou un cylindre présentant une densité supérieure ou égale à celle d'au moins un agent spécifique contenu dans le tube. L'élément lesté est configuré pour prendre au moins une deuxième position postérieure au prélèvement de l'échantillon, dans laquelle l'élément lesté est libre dans le tube de réception, l'élément lesté étant poussé à l'intérieur du tube lors dudit prélèvement.

Un tel dispositif de prélèvement et/ou dispositif de stockage présente les mêmes avantages que le système de prélèvement décrit précédemment. Ils ne sont pas détaillés plus amplement.

L'invention concerne par ailleurs un procédé de fabrication d'un dispositif de prélèvement ou d'un dispositif de stockage tels que décrits ci-dessus, comprenant une étape d'insertion d'un élément lesté dans le dispositif de prélèvement ou dans le dispositif de stockage. Un tel élément lesté est configuré pour prendre au moins deux positions, dont une première position antérieure au prélèvement de l'échantillon, dans laquelle l'élément lesté est maintenu dans le dispositif de prélèvement ou dans le dispositif de stockage, et une deuxième position postérieure au prélèvement de l'échantillon, dans laquelle l'élément lesté est libre dans le tube, l'élément lesté étant poussé à l'intérieur du tube lors du prélèvement, l'élément lesté étant une sphère ou un cylindre présentant une densité supérieure ou égale à celle d'au moins un agent spécifique contenu dans le tube de réception.

En particulier, lorsque l'élément lesté est inséré dans le dispositif de stockage, l'étape d'insertion met en oeuvre une obturation d'un orifice d'entrée du tube.

A nouveau, les avantages de ce procédé de fabrication sont les mêmes que ceux présentés en relation avec le système de prélèvement. Ils ne sont pas détaillés plus amplement. En particulier, un tel procédé de fabrication est simple à mettre en oeuvre, et aisément industrialisable.

Selon une variante, un tel procédé de fabrication comprend une première étape d'insertion, dans le tube, d'une tête de tube comprenant un capuchon percé d'une ouverture centrale et une collerette, destinée à prendre appui sur le rebord du tube, et une deuxième étape d'insertion, dans la tête de tube, de l'élément lesté, de façon que, dans la première position, l'élément lesté obture l'ouverture centrale de la tête de tube.

L'insertion de l'élément lesté dans la tête de tube après que cette dernière soit placée dans le tube permet avantageusement de diminuer la pression du tube en comprimant un volume d'air plus faible.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- la figure 1, présentée en relation avec l'art antérieur, illustre un dispositif de prélèvement d'un échantillon de tissu selon l'art antérieur ;
- les figures 2A à 2C illustrent un premier mode de réalisation de l'invention, selon lequel l'élément lesté est, dans sa première position, maintenu dans un tube de réception d'un dispositif de stockage ;
- les figures 3A à 3C présentent un deuxième mode de réalisation de l'invention, selon lequel l'élément lesté est, dans sa première position, maintenu dans une tête de tube d'un tube de réception d'un dispositif de stockage ;
- les figures 4A à 4C illustrent un troisième mode de réalisation de l'invention, selon lequel l'élément lesté est, dans sa première position, maintenu dans un dispositif de prélèvement ;
- la figure 5 illustre un système de prélèvement selon le deuxième mode de réalisation ;
- la figure 6 présente les principales étapes mises en oeuvre par un procédé de fabrication selon l'invention.

### 5. Description d'un mode de réalisation de l'invention

### 5.1 Principe général

Le principe général de l'invention repose sur l'utilisation d'un élément lesté, combinée à l'utilisation d'un dispositif de prélèvement et/ou de stockage, pour former un système de prélèvement d'un échantillon de tissus animal particulièrement astucieux. On entend ici par élément lesté, un élément ayant une certaine masse.

L'invention propose ainsi un nouveau système de prélèvement dans lequel est inséré l'élément lesté qui peut prendre au moins deux positions.

En particulier, dans une première position préalable au prélèvement de l'échantillon, l'élément lesté est maintenu en position dans le dispositif de prélèvement ou le dispositif de stockage. Avantageusement, lorsqu'il est maintenu dans le dispositif de stockage, l'élément lesté obture l'orifice d'entrée du tube et peut assurer une fonction de bouchon du tube de réception.

Dans une deuxième position, ultérieure au prélèvement de l'échantillon, l'élément lesté est introduit à l'intérieur du tube en même temps que l'échantillon, et libéré à l'intérieur du tube. Il peut donc assurer une fonction d'agitateur ou mélangeur, de lest, ou de pilon.

Ainsi, dans un mode de réalisation avantageux, l'élément lesté comprend un élément magnétique permettant d'assurer la fonction d'agitateur dans le tube de réception, lorsqu'il prend sa deuxième position. L'élément lesté permet alors de faciliter le mélange dans le tube, notamment lorsque des réactifs sont ajoutés dans le tube, lors de l'analyse de l'échantillon.

On note toutefois que l'élément lesté est préférentiellement réalisé dans un matériau neutre et inerte, c'est-à-dire qui ne produit aucune réaction chimique ou physique avec des agents spécifiques/réactifs introduits dans le tube de réception antérieurement ou postérieurement au prélèvement.

Par ailleurs, l'élément lesté prend la forme d'une sphère ou un cylindre, présentant une section circulaire, pour s'adapter au mieux à la forme usuelle des tubes de réception. Ainsi, il présente de manière préférentielle un diamètre sensiblement égal (ou très légèrement inférieur) au diamètre de l'orifice d'entrée du tube. De manière préférentielle, son diamètre est également supérieur au diamètre de l'orifice d'entrée d'une pipette utilisée pour extraire l'échantillon du tube ou une quantité d'agent spécifique.

### 5.2 Premier mode de réalisation

Les figures 2A à 2C illustrent un premier mode de réalisation de l'invention selon lequel l'élément lesté est présent dans le dispositif de stockage, et maintenu directement dans le tube de réception, préalablement au prélèvement.

Plus précisément, les figures 2A à 2C présentent une vue en coupe d'un système de prélèvement selon ce premier mode de réalisation, qui comprend :
- un dispositif de prélèvement comprenant un élément de coupe 31 destiné à découper un échantillon de tissu animal, et
- un dispositif de stockage comprenant un tube de réception 21 destiné à recevoir l'échantillon et un élément lesté 22, par exemple une sphère creuse ou pleine, également appelée bille.

On considère par exemple que la forme du tube de réception 21 est compatible avec un support de tubes d'échantillon de type microplaque (« rack » en anglais), comprenant par exemple 24,48 ou 96 positions.

Selon ce premier exemple de réalisation, on considère que le tube de réception 21 contient, préalablement au prélèvement, au moins un agent spécifique 23 sous forme de gel, liquide, poudre, billes ou mousse permettant notamment d'améliorer la conservation de l'échantillon ou de le préparer à de futures analyses en laboratoire. Bien entendu, le tube de réception peut être vide avant le prélèvement, et un tel agent spécifique peut être introduit après le prélèvement, par la personne réalisant le prélèvement ou une personne analysant l'échantillon prélevé par exemple.

On décrit dans un premier temps, en relation avec la figure 2A, la première position de l'élément lesté 22, préalablement au prélèvement.

Dans cette première position, l'élément lesté 22 est maintenu en position dans la partie supérieure du dispositif de stockage, à l'entrée du tube de réception 21, par des ergots 211 présents sur la surface interne du tube, et obture l'orifice d'entrée du tube. Ainsi, l'élément lesté 22 sert de bouchon au tube de réception 21, et l'étanchéité de l'agent spécifique 23 présent dans le tube de réception 21 est assurée.

On décrit désormais, en relation avec la figure 2B, la deuxième position de l'élément lesté 22, après prélèvement de l'échantillon.

Comme illustré sur cette figure, lors du prélèvement, l'élément de coupe 31 du dispositif de prélèvement, éventuellement solidarisé à un support 32, découpe la peau de l'animal, pousse l'élément lesté 22 à l'intérieur du tube 21 et vient s'insérer au moins partiellement dans le tube 21. L'arête coupante de l'élément de coupe 31, qui présente un diamètre légèrement inférieur à celui de l'orifice d'entrée du tube de réception de manière à pouvoir s'insérer dans le tube 21, vient alors en butée contre les ergots 211 présents sur la surface intérieure du tube. Pour ce faire, on considère que l'élément lesté 22 et/ou les parois du tube 21 peuvent subir une légère déformation.

On rappelle que le support 32, qui peut être réalisé en matière plastique, est classiquement monté à l'extrémité d'un pointeau solidarisé à un des mors d'une pince de prélèvement, de façon amovible. Il se présente sous la forme d'une surface de révolution, de même axe que l'élément de coupe 31. Selon une variante, l'élément de coupe 31 et le support 32 sont formés d'un seul tenant, par exemple en plastique ou en métal. On considère alors que l'élément de coupe 31 et le support 32 forment une seule pièce, qui est « monobloc ».

L'élément lesté 22 se trouve alors libéré dans le tube 21, entre le fond du tube et l'échantillon 33. En particulier, comme l'élément lesté 22 présente une densité supérieure à celle de l'agent spécifique 23, il coule au fond du tube de réception 21 lorsque celui-ci est immobile. L'élément lesté 22, dans sa deuxième position, peut alors assurer une fonction d'agitateur ou mélangeur. De plus, l'élément lesté peut servir d'indicateur visuel pour indiquer que le prélèvement a été correctement effectué, notamment s'il présente une couleur vive.

La figure 2C illustre une variante de réalisation de l'élément lesté.

Selon cette variante, l'élément lesté 22 possède des moyens d'accroche 221 de l'échantillon 33, permettant de solidariser l'échantillon à l'élément lesté lors du prélèvement et de l'entraîner dans le fond du tube de réception 21. Cette variante permet ainsi d'assurer une meilleure préservation et/ou un meilleur traitement de l'échantillon, par l'agent spécifique 23. En effet, de cette manière, l'échantillon 33 a une forte probabilité d'être recouvert par l'agent spécifique (sous forme liquide par exemple), sans pour autant nécessiter une quantité importante de cet agent.

Différentes formes sont envisagées pour ces moyens d'accroche 221 de l'échantillon.

Ainsi, comme illustré en figure 2C, de tels moyens d'accrochage 221 prennent la forme d'un harpon. Selon d'autres variantes non illustrées, de tels moyens d'accrochage prennent la forme d'une pointe, d'une aiguille, d'un crochet, etc.

### 53 Deuxième mode de réalisation

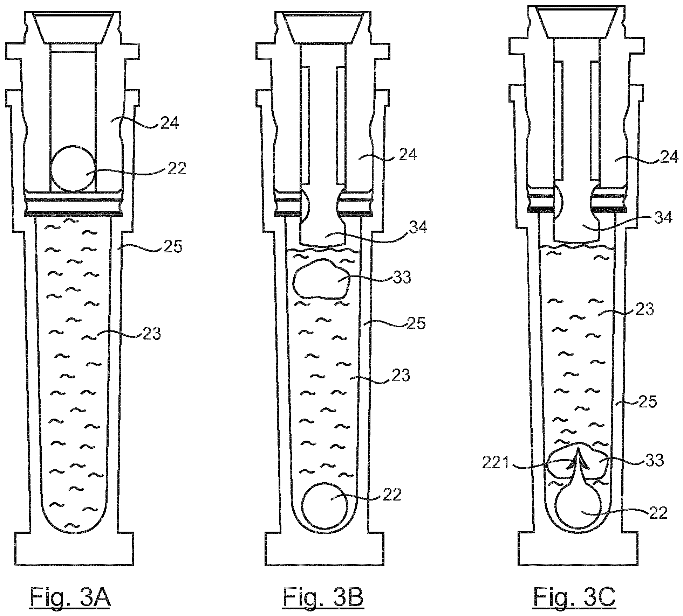

Les figures 3A à 3C illustrent un deuxième mode de réalisation de l'invention selon lequel l'élément lesté est présent dans le dispositif de stockage, et maintenu dans une tête de tube du tube de réception, préalablement au prélèvement.

Plus précisément, les figures 3A à 3C présentent une vue en coupe d'un dispositif de stockage selon ce deuxième mode de réalisation, qui comprend un tube de réception 25 destiné à recevoir l'échantillon, une tête de tube 24 et un élément lesté 22, par exemple une sphère creuse ou pleine, également appelée bille.

Une telle tête de tube 24 est solidarisée à l'entrée du tube de réception 25, par exemple par clippage ou emboîtement. Elle peut être réalisée en matière flexible, notamment en caoutchouc, matière plastique, ou autre, pour faciliter son insertion dans le col du tube.

Plus précisément, la tête de tube 24 se présente sous la forme d'un capuchon percé d'une ouverture centrale, de diamètre suffisant pour permettre l'insertion de l'élément lesté 22. L'utilisation de matière flexible pour ce capuchon permet aussi de faciliter la mise en place de l'élément lesté. La tête de tube 24 présente également une collerette, pouvant prendre appui sur le rebord du tube de réception 25. L'utilisation d'une telle collerette permet notamment de faciliter la pose et l'enlèvement du capuchon. La collerette définit également une surface de butée sur laquelle l'arête coupante d'un élément de coupe peut prendre appui lors du prélèvement, afin de découper plus facilement l'échantillon de tissus. On note que le tube peut également être en matière plus ou moins élastique. Il s'avère néanmoins souhaitable que la tête de tube soit suffisamment rigide pour servir de surface d'appui pour une bonne découpe de l'échantillon.

On considère également à nouveau que la forme du tube de réception 25 est compatible avec un support de tubes d'échantillon de type microplaque, comprenant par exemple 24, 48 ou 96 positions.

On décrit dans un premier temps, en relation avec la figure 3A, la première position de l'élément lesté 22, préalablement au prélèvement.

Dans cette première position, l'élément lesté 22 est introduit dans la tête de tube 24, de manière à boucher l'ouverture centrale de la tête de tube 24 du tube 25 qui peut, comme décrit en relation avec le premier mode de réalisation, contenir ou non un agent spécifique 23. Par exemple, l'élément lesté est emboîté à force dans la tête de tube 24, et maintenu dans la tête de tube grâce aux propriétés élastiques de la matière utilisée pour la tête de tube. A nouveau, comme décrit en relation avec le premier mode de réalisation, l'élément lesté 22 sert de bouchon ou couvercle au tube de réception 25 dans cette première position.

On décrit désormais, en relation avec la figure 3B, la deuxième position de l'élément lesté 22, après prélèvement de l'échantillon.

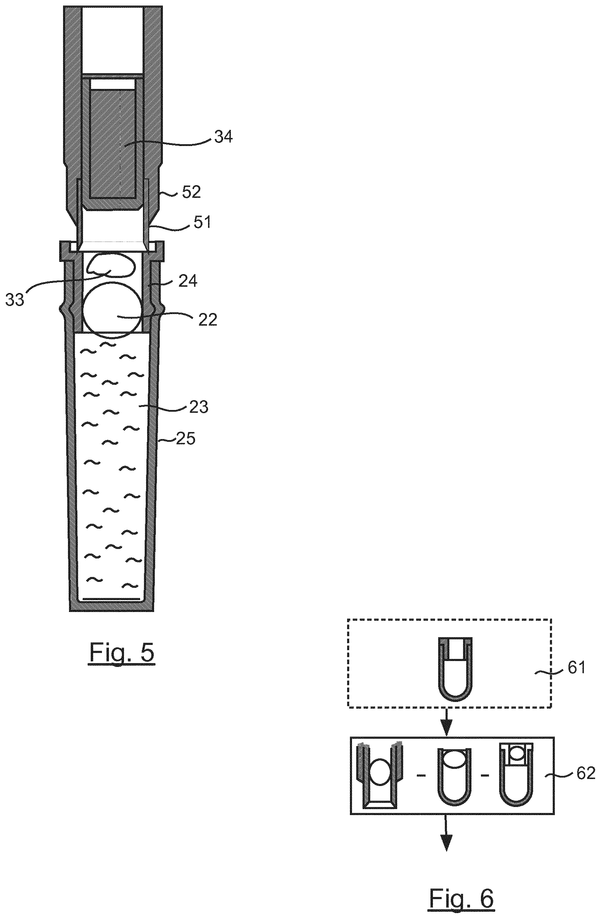

A titre d'exemple, et comme illustré en figure 5, on considère que le prélèvement met en oeuvre des moyens de prélèvement tels que décrits dans la demande de brevet WO2010/066475 précitée, comprenant deux éléments distincts mobiles l'un par rapport à l'autre, dont un élément de coupe 51 permettant de découper les tissus de l'animal et un élément poussoir 34 permettant de pousser l'échantillon ainsi découpé dans le dispositif de stockage. Bien entendu, un dispositif de prélèvement classique pourrait également être utilisé avec un dispositif de stockage selon ce deuxième mode de réalisation.

Comme illustré sur cette figure 5, lors du prélèvement, l'élément de coupe 51, éventuellement solidarisé à un support 52, découpe dans un premier temps la peau de l'animal.

Dans un deuxième temps, illustré en figure 3B, l'élément poussoir 34 vient pousser l'échantillon 33 ainsi découpé dans le canal de la tête de tube 24, à travers son ouverture centrale, pousser l'élément lesté 22 à l'intérieur du tube 25 et s'insérer au moins partiellement dans la tête de tube 24. L'élément poussoir 34 est alors solidarisé à la tête de tube 24 par emboîtement à force par exemple, et obture ainsi hermétiquement ou quasi-hermétiquement le tube 25.

L'élément lesté 22 se trouve alors libéré dans le tube 25, entre le fond du tube et l'échantillon 33, dans sa deuxième position. En particulier, comme décrit en relation avec le premier mode de réalisation, comme l'élément lesté 22 présente une densité supérieure à celle de l'agent spécifique 23, il coule au fond du tube de réception 25 lorsque celui-ci est immobile. L'élément lesté 22, dans sa deuxième position, peut alors assurer une fonction d'agitateur ou mélangeur.

Une fois l'échantillon prélevé et l'élément poussoir 34 au moins partiellement inséré dans l'ouverture centrale de la tête de tube 24, il est possible de retirer d'un seul bloc la tête de tube 24 et l'élément poussoir 34 pour analyser l'échantillon, par préhension de la collerette. Cette étape d'ouverture du tube de réception 25, permettant de décapsuler la tête de tube de façon à ne laisser que l'échantillon de tissu dans le tube 25, peut ainsi être automatisée.

La figure 3C illustre une variante de réalisation de l'élément lesté 22.

Selon cette variante, comme décrit en relation avec la figure 2C, l'élément lesté 22 possède des moyens d'accroche 221 de l'échantillon 33, prenant la forme d'un harpon, d'une pointe, d'une aiguille, d'un crochet, etc, permettant de solidariser l'échantillon à l'élément lesté lors du prélèvement et de l'entraîner dans le fond du tube de réception 51.

Cette variante permet ainsi d'assurer une meilleure préservation et/ou un meilleur traitement de l'échantillon, par l'agent spécifique 23, prévu dans le tube préalablement au prélèvement ou introduit postérieurement.

### 5.4 Troisième mode de réalisation

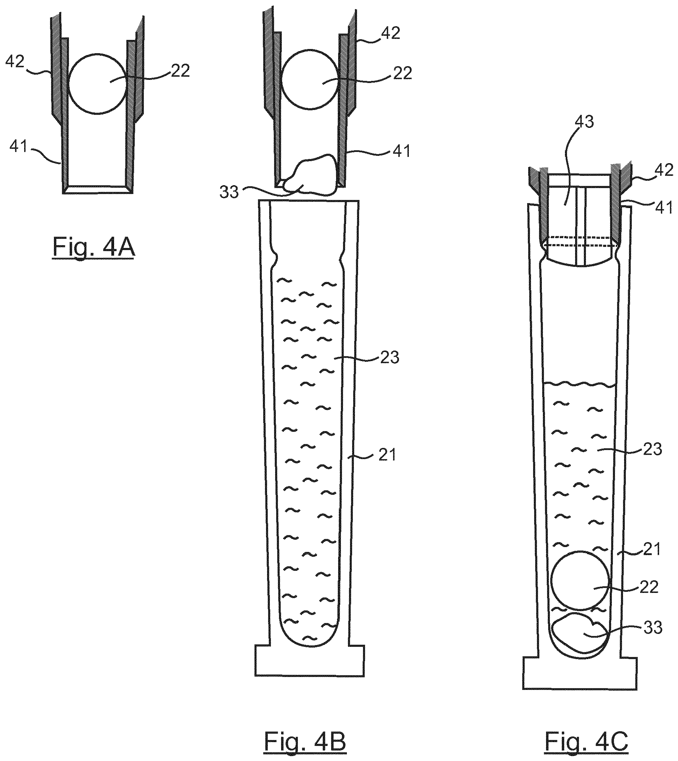

Les figures 4A à 4C illustrent un troisième mode de réalisation de l'invention, selon lequel l'élément lesté est présent dans le dispositif de prélèvement, et maintenu dans l'élément de coupe ou dans un support de l'élément de coupe, préalablement au prélèvement.

Plus précisément, les figures 4A à 4C présentent une vue en coupe d'un système de prélèvement selon ce troisième mode de réalisation, qui comprend :
- un dispositif de prélèvement comprenant un élément de coupe 41 destiné à découper un échantillon de tissu animal, et un élément lesté 22, par exemple une sphère creuse ou pleine, également appelée bille;
- un dispositif de stockage comprenant un tube de réception 21 destiné à recevoir l'échantillon.

On décrit dans un premier temps, en relation avec la figure 4A, la première position de l'élément lesté 22, préalablement au prélèvement.

Dans cette première position, l'élément lesté 22 est introduit dans le dispositif de prélèvement, par exemple à l'intérieur de l'élément de coupe 41 ou d'un support 42 de l'élément de coupe (qui présentent généralement une forme de révolution).

Par exemple, l'élément lesté est emboîté à force dans le dispositif de prélèvement, et maintenu en position grâce aux propriétés mécaniques de l'élément lesté ou du dispositif de prélèvement. Selon une variante, non illustrée, l'élément lesté 22 est maintenu dans sa première position par des ergots présents sur la surface interne de l'élément de coupe 41 ou de son support 42.

Comme illustré en relation avec la figure 4B, lors de l'opération de prélèvement, l'élément de coupe 41 découpe dans un premier temps la peau de l'animal.

Dans un deuxième temps, illustré en figure 4C, l'échantillon est poussé hors du dispositif de prélèvement, par l'intermédiaire de l'élément lesté 22. En d'autres termes, un extracteur 43, par exemple de type aiguille, tige, élément poussoir 34 tel que décrit en relation avec les figures 3A à 3C, etc, coulissant dans l'élément de coupe 41, pousse l'élément lesté 22 en direction du tube 21, l'élément lesté entraînant à son tour l'échantillon 33 dans le tube d'échantillon 21. L'élément lesté 22 se trouve alors libéré dans le tube 21, dans sa deuxième position.

Du fait des propriétés spécifiques de l'élément lesté 22, et notamment de son poids, l'élément lesté 22 est entraîné vers le fond du tube 21, et entraîne avec lui l'échantillon 33. L'élément lesté 22, dans sa deuxième position, peut alors assurer une fonction de lest pour l'échantillon 33, ou de pilon permettant d'écraser ou d'effriter l'échantillon afin de faciliter les analyses ultérieures.

On note que selon une variante, un tel élément lesté 22 peut être équipé de moyens d'accroche de l'échantillon, tels qu'illustrés en figures 2C et 3C, permettant de solidariser l'échantillon à l'élément lesté.

L'extracteur 43 peut être actionné manuellement ou par l'intermédiaire d'un outil de prélèvement à double mouvement (l'un actionnant l'élément de coupe, l'autre l'extracteur).

Ce troisième mode de réalisation permet de s'assurer que l'échantillon 33 se situe entre le fond du tube 21 et l'élément lesté 22 après le prélèvement. On assure ainsi une meilleure préservation et/ou un meilleur traitement de l'échantillon par un agent spécifique 23, qui peut être prévu dans le tube préalablement au prélèvement ou introduit postérieurement au prélèvement.

Bien entendu, ce troisième mode de réalisation peut être mis en oeuvre avec un tube de réception comprenant ou non une tête de tube. De la même façon, il peut être mis en oeuvre dans un dispositif de prélèvement tel que décrit dans la demande de brevet WO2010/066475 précitée, comprenant deux éléments distincts mobiles l'un par rapport à l'autre.

### 5-5 Procédé de fabrication

On décrit ci-après, en relation avec la figure 6, les principales étapes de fabrication d'un système de prélèvement comprenant un dispositif de prélèvement et/ou un dispositif de stockage selon les modes de réalisation décrits précédemment.

Selon ces modes de réalisation, un tel procédé comprend une étape d'insertion 62 d'un élément lesté dans le dispositif de prélèvement ou dans le dispositif de stockage, ledit élément lesté étant configuré pour prendre les deux positions décrites plus haut.

Une telle étape peut être précédée d'une étape d'insertion 61 d'une tête de tube dans le tube de réception. Dans ce cas, l'élément lesté peut être inséré dans la tête de tube une fois que la tête de tube a elle-même été insérée dans le tube de réception, afin de créer moins de pression dans le tube de réception en comprimant un volume d'air plus faible.

## Revendications

1. Système de prélèvement d'au moins un échantillon (33) de tissu animal, comprenant :
- un dispositif de prélèvement comprenant au moins un élément de coupe (31 ; 41 ; 51) destiné à découper un échantillon de tissu animal, et
- un dispositif de stockage comprenant un tube de réception (21 ; 25) destiné à recevoir ledit échantillon,
**caractérisé en ce que** ledit système comprend un élément lesté (22) configuré pour prendre au moins deux positions, dont une première position antérieure au prélèvement dudit échantillon, dans laquelle ledit élément lesté (22) est maintenu dans ledit dispositif de prélèvement ou dans ledit dispositif de stockage, et une deuxième position postérieure au prélèvement dudit échantillon, dans laquelle ledit élément lesté (22) est libre dans ledit tube, ledit élément lesté (22) étant poussé à l'intérieur dudit tube lors dudit prélèvement,
et **en ce que** ledit élément lesté (22) est une sphère ou un cylindre présentant une densité supérieure ou égale à celle d'au moins un agent spécifique (23) contenu dans ledit tube (21 ; 25).

2. Système de prélèvement selon la revendication 1, **caractérisé en ce que** ledit au moins un agent spécifique appartient au groupe comprenant :
- un agent conservateur ;
- un agent dessiccant ;
- un agent réactif ;
- un agent de préparation dudit échantillon.

3. Système de prélèvement selon l'une quelconque des revendications 1 et 2, **caractérisé en ce que** ledit élément lesté (22) présente une couleur spécifique.

4. Système de prélèvement selon l'une quelconque des revendications 1 à 3, **caractérisé en ce que** ledit élément lesté (22) comprend des moyens d'accroche (221) dudit échantillon, permettant de solidariser ledit échantillon (33) audit élément lesté (22).

5. Système de prélèvement selon la revendication 4, **caractérisé en ce que** lesdits moyens d'accroche (221) comprennent un élément appartenant au groupe comprenant :
- une pointe ;
- une aiguille ;
- un crochet ;
- un harpon.

6. Système de prélèvement selon l'une quelconque des revendications 1 à 5 **caractérisé en ce que** ledit élément lesté (22) est inerte.

7. Système de prélèvement selon l'une quelconque des revendications 1 à 6 **caractérisé en ce que** ledit élément lesté (22) comprend un aimant.

8. Système de prélèvement selon l'une quelconque des revendications 1 à 7, **caractérisé en ce que**, dans ladite première position, ledit élément lesté (22) est maintenu dans la partie supérieure dudit dispositif de stockage et obture un orifice d'entrée dudit tube (21 ; 25).

9. Système de prélèvement selon la revendication 8 **caractérisé en ce que** ledit dispositif de stockage comprend une tête de tube (24) comprenant un capuchon percé d'une ouverture centrale et une collerette,
et **en ce que**, dans ladite première position, ledit élément lesté (22) obture ladite ouverture centrale.

10. Système de prélèvement selon l'une quelconque des revendications 1 à 7, **caractérisé en ce que**, dans ladite première position, ledit élément lesté (22) est maintenu dans ledit dispositif de prélèvement, à l'intérieur dudit élément de coupe (41).

11. Système de prélèvement selon l'une quelconque des revendications 1 à 10, **caractérisé en ce que** ledit dispositif de prélèvement comprend également un élément poussoir (34) mobile par rapport audit élément de coupe, permettant de pousser ledit échantillon dans ledit dispositif de stockage après découpe de l'échantillon par ledit élément de coupe,
ledit élément lesté étant poussé par ledit élément poussoir à l'intérieur dudit tube lors dudit prélèvement.

12. Dispositif de prélèvement d'au moins un échantillon de tissu animal comprenant au moins un élément de coupe (31 ; 41 ; 51) destiné à découper un échantillon de tissu animal,
apte à coopérer avec un dispositif de stockage comprenant un tube de réception (21 ; 25) destiné à recevoir ledit échantillon,
**caractérisé en ce que** ledit dispositif de prélèvement comprend un élément lesté (22) dans une première position antérieure au prélèvement dudit échantillon, dans laquelle ledit élément lesté (22) est maintenu dans ledit dispositif de prélèvement,
et **en ce que** ledit élément lesté (22) est une sphère ou un cylindre présentant une densité supérieure ou égale à celle d'au moins un agent spécifique (23) contenu dans ledit tube (21 ; 25),ledit élément lesté étant configuré pour prendre au moins une deuxième position postérieure au prélèvement dudit échantillon, dans laquelle ledit élément lesté (22) est libre dans ledit tube de réception (21 ; 25),
ledit élément lesté (22) étant poussé à l'intérieur dudit tube lors dudit prélèvement.

13. Dispositif de stockage d'au moins un échantillon de tissu animal comprenant un tube de réception (21 ; 25) destiné à recevoir un échantillon de tissu animal,
apte à coopérer avec un dispositif de prélèvement comprenant au moins un élément de coupe (31 ; 41 ; 51) destiné à découper ledit échantillon,
**caractérisé en ce que** ledit dispositif de stockage comprend un élément lesté (22) dans une première position antérieure au prélèvement dudit échantillon, dans laquelle ledit élément lesté (22) est maintenu dans ledit dispositif de stockage,
et **en ce que** ledit élément lesté (22) est une sphère ou un cylindre présentant une densité supérieure ou égale à celle d'au moins un agent spécifique (23) contenu dans ledit tube (21 ; 25),ledit élément lesté étant configuré pour prendre au moins une deuxième position postérieure au prélèvement dudit échantillon, dans laquelle ledit élément lesté (22) est libre dans ledit tube de réception (21 ; 25),
ledit élément lesté (22) étant poussé à l'intérieur dudit tube lors dudit prélèvement.

14. Procédé de fabrication d'un dispositif de prélèvement selon la revendication 12 ou d'un dispositif de stockage selon la revendication 13,
**caractérisé en ce qu'**il comprend une étape d'insertion d'un élément lesté (22) dans ledit dispositif de prélèvement ou dans ledit dispositif de stockage,
ledit élément lesté (22) étant une sphère ou un cylindre présentant une densité supérieure ou égale à celle d'au moins un agent spécifique (23) contenu dans ledit tube (21 ; 25),
ledit élément lesté étant configuré pour prendre au moins deux positions, dont une première position antérieure au prélèvement dudit échantillon, dans laquelle ledit élément lesté (22) est maintenu dans ledit dispositif de prélèvement ou dans ledit dispositif de stockage, et une deuxième position postérieure au prélèvement dudit échantillon, dans laquelle ledit élément lesté (22) est libre dans ledit tube, ledit élément lesté (22) étant poussé à l'intérieur dudit tube lors dudit prélèvement.

## Patentansprüche

1. System zur Entnahme mindestens einer Tiergewebeprobe (33), aufweisend:
- eine Entnahmevorrichtung, die mindestens ein Schneidelement (31; 41; 51) enthält, das dazu bestimmt ist, eine Tiergewebeprobe auszuschneiden, und
- eine Lagervorrichtung, die ein Aufnahmerohr (21; 25) enthält, das dazu bestimmt ist, die Probe aufzunehmen,
**dadurch gekennzeichnet, dass** das System ein beschwertes Element (22) enthält, das dazu konfiguriert ist, mindestens zwei Stellungen einzunehmen, darunter eine erste Stellung vor der Entnahme der Probe, in der das beschwerte Element (22) in der Entnahmevorrichtung oder in der Lagervorrichtung gehalten wird, und eine zweite Stellung nach der Entnahme der Probe, in der das beschwerte Element (22) im Rohr frei ist, wobei das beschwerte Element (22) bei der Entnahme ins Innere des Rohrs gedrückt wird,
und dass das beschwerte Element (22) eine Kugel oder ein Zylinder mit einer höheren oder gleichen Dichte relativ zu derjenigen mindestens eines im Rohr (21; 25) enthaltenen spezifischen Mittels (23) ist.

2. Entnahmesystem nach Anspruch 1, **dadurch gekennzeichnet, dass** das mindestens eine spezifische Mittel zu der Gruppe gehört, die enthält:
- ein Konservierungsmittel;
- ein Trocknungsmittel;
- ein Reaktionsmittel;
- ein Mittel zur Präparationder Probe.

3. Entnahmesystem nach einem der Ansprüche 1 und 2, **dadurch gekennzeichnet, dass** das beschwerte Element (22) eine spezifische Farbe hat.

4. Entnahmesystem nach einem der Ansprüche 1 bis 3, **dadurch gekennzeichnet, dass** das beschwerte Element (22) Befestigungseinrichtung(221) der Probe enthält, die es ermöglichen, die Probe (33) fest mit dem beschwerten Element (22) zu verbinden.

5. Entnahmesystem nach Anspruch 4, **dadurch gekennzeichnet, dass** die Befestigungseinrichtung(221) ein Element aufweisen, das zu der Gruppe umfassend:
- eine Spitze;
- eine Nadel;
- einen Haken;
- einen Widerhaken
gehört.

6. Entnahmesystem nach einem der Ansprüche 1 bis 5 **dadurch gekennzeichnet, dass** das beschwerte Element (22) inert ist.

7. Entnahmesystem nach einem der Ansprüche 1 bis 6 **dadurch gekennzeichnet, dass** das beschwerte Element (22) einen Magnet aufweist.

8. Entnahmesystem nach einem der Ansprüche 1 bis 7, **dadurch gekennzeichnet, dass** in der ersten Stellung das beschwerte Element (22) im oberen Teil der Lagervorrichtung gehalten wird und eine Eingangsöffnung des Rohrs (21; 25) verschließt.

9. Entnahmesystem nach Anspruch 8, **dadurch gekennzeichnet, dass** die Lagervorrichtung einen Rohrkopf (24) aufweist, der eine Kappe, in die eine zentrale Öffnung gebohrt ist, und einen Kragen enthält,
und dass in der ersten Stellung das beschwerte Element (22) die zentrale Öffnung verschließt.

10. Entnahmesystem nach einem der Ansprüche 1 bis 7, **dadurch gekennzeichnet, dass** in der ersten Stellung das beschwerte Element (22) in der Entnahmevorrichtung im Inneren des Schneidelements (41) gehalten wird.

11. Entnahmesystem nach einem der Ansprüche 1 bis 10, **dadurch gekennzeichnet, dass** die Entnahmevorrichtung ebenfalls ein bezüglich des Schneidelements bewegliches Drückelement (34) aufweist, das es ermöglicht, die Probe nach dem Ausschneiden der Probe mittels des Schneidelements in die Lagervorrichtung zu drücken,
wobei das beschwerte Element bei der Entnahme vom Drückelement ins Innere des Rohrs gedrückt wird.

12. Vorrichtung zur Entnahme mindestens einer Tiergewebeprobe, die mindestens ein Schneidelement (31; 41; 51) aufweist, das dazu bestimmt ist, eine Tiergewebeprobe auszuschneiden,
die dazu geeignet ist, mit einer Lagervorrichtung zusammenzuwirken, die ein Aufnahmerohr (21; 25) aufweist, das dazu bestimmt ist, die Probe aufzunehmen,
**dadurch gekennzeichnet, dass** die Entnahmevorrichtung ein beschwerte Element (22) in einer ersten Stellung vor der Entnahme der Probe aufweist, in der das beschwerte Element (22) in der Entnahmevorrichtung gehalten wird,
und dass das beschwerte Element (22) eine Kugel oder ein Zylinder mit einer höheren oder gleichen Dichte relativ zu derjenigen mindestens eines im Rohr (21; 25) enthaltenen spezifischen Mittels (23) ist, wobei das beschwerte Element dazu konfiguriert ist, mindestens eine zweite Stellung nach der Entnahme der Probe einzunehmen, in der das beschwerte Element (22) im Aufnahmerohr (21; 25) frei ist,
wobei das beschwerte Element (22) bei der Entnahme ins Innere des Rohrs gedrückt wird.

13. Vorrichtung zum Lagern mindestens einer Tiergewebeprobe, die ein Aufnahmerohr (21; 25) enthält, das dazu bestimmt ist, eine Tiergewebeprobe aufzunehmen,
die dazu geeignet ist, mit einer Entnahmevorrichtung zusammenzuwirken, die mindestens ein Schneidelement (31; 41; 51) aufweist, das dazu bestimmt ist, die Probe auszuschneiden,
**dadurch gekennzeichnet, dass** die Lagervorrichtung ein beschwerte Element (22) in einer ersten Stellung vor der Entnahme der Probe aufweist, in der das beschwerte Element (22) in der Lagervorrichtung gehalten wird,
und dass das beschwerte Element (22) eine Kugel oder ein Zylinder mit einer höheren oder gleichen Dichte relativ zu derjenigen mindestens eines im Rohr (21; 25) enthaltenen spezifischen Mittels (23) ist, wobei das beschwerte Element dazu konfiguriert ist, mindestens eine zweite Stellung nach der Entnahme der Probe einzunehmen, in der das beschwerte Element (22) im Aufnahmerohr (21; 25) frei ist,
wobei das beschwerte Element (22) bei der Entnahme ins Innere des Rohrs gedrückt wird.

14. Verfahren zur Herstellung einer Entnahmevorrichtung nach Anspruch 12 oder einer Lagervorrichtung nach Anspruch 13,
**dadurch gekennzeichnet, dass** es einen Schritt der Einführung eines beschwerten Elements (22) in die Entnahmevorrichtung oder in die Lagervorrichtung umfasst,
wobei das beschwerte Element (22) eine Kugel oder ein Zylinder mit einer höheren oder gleichen Dichte relativ zu derjenigen mindestens eines im Rohr (21; 25) enthaltenen spezifischen Mittels (23) ist,
wobei das beschwerte Element dazu konfiguriert ist, mindestens zwei Stellungen einzunehmen, darunter eine erste Stellung vor der Entnahme der Probe, in der das beschwerte Element (22) in der Entnahmevorrichtung oder in der Lagervorrichtung gehalten wird, und eine zweite Stellung nach der Entnahme der Probe, in der das beschwerte Element (22) im Rohr frei ist, wobei das beschwerte Element (22) bei der Entnahme ins Innere des Rohrs gedrückt wird.

## Claims

1. System for collecting at least one sample (33) of animal tissue, comprising:
- a device for collecting comprising at least one cutting element (31; 41; 51) designed for cutting out a sample of animal tissue, and
- a device for storing comprising a receiving tube (21; 25) designed for receiving said sample,
wherein said system also comprises a weighted element (22) configured to take at least two positions, comprising one first position prior to the collecting of said sample, in which said weighted element (22) is held in said device for collecting or in said device for storing, and a second position subsequent to the collecting of said sample, in which said weighted element (22) (22) is released in said tube, said weighted element (22) being pushed into said tube during said collecting,
and wherein said weighted element (22) is a sphere or a cylinder having a density greater than or equal to that of at least one specific agent (23) contained in said tube (21; 25).

2. System for collecting according to claim 1, wherein said at least one specific agent belongs to the group comprising:
- a preserving agent,
- a desiccant,
- a reagent,
- an agent for preparing said sample.

3. System for collecting according to any one of the claims 1 and 2, wherein said weighted element (22) has a specific color.

4. System for collecting according to any one of the claims 1 to 3, wherein said weighted element (22) comprises means (221) for hooking on to said sample, enabling the sample (33) to be fixedly attached to said weighted element (22)

5. System for collecting according to claim 4, wherein said means for hooking (221) comprise an element belonging to the group comprising:
- a spike;
- a needle;
- a hook;
- a harpoon.

6. System for collecting according to any one of the claims 1 to 5, wherein said weighted element (22) is inert.

7. System for collecting according to any one of the claims 1 to 6 wherein said weighted element (22) comprises a magnet.

8. System for collecting according to any one of the claims 1 to 7, wherein, in said first position, said weighted element (22) is held in the upper part of said device for storing, and closes off an inlet orifice of said tube (21; 25).

9. System for collecting according to claim 8 **characterized in** said device for storing comprises a tube head (24) comprising a hood, pierced with a central aperture, and a flange,
and wherein, in said first position, said weighted element (22) closes off said central aperture.

10. System for collecting according to any one of the claims 1 to 7, wherein, in said first position, said weighted element (22) is held in said device for collecting, inside said cutting element (41).

11. System for collecting according to any one of the claims 1 to 10, wherein said device for collecting also comprises a pusher element (34) that is mobile relative to said cutting element, configured to push said sample into said device for storing after the sample has been cut out by said cutting element,
said weighted element being pushed by said pusher element into said tube during said collecting.

12. Device for collecting at least one sample of animal tissue comprising at least one cutting element (31; 41; 51) for cutting out a sample of animal tissue,
designed to cooperate with a device for storing comprising a receiving tube (21; 25) designed for receiving said sample.
wherein said device for collecting comprises a weighted element (22) in a first position prior to the collecting of said sample, in which said weighted element (22) is held in said device for collecting,
and wherein said weighted element (22) is a sphere or a cylinder having a density greater than or equal to that of at least one specific agent (23) contained in said tube (21; 25), said weighted element being configured to take at least one second position subsequently to the collecting of said sample, in which said weighted element (22) is released in said receiving tube (21; 25), the weighted element (22) being pushed into the tube during said collecting (21, 23).

13. Device for storing at least one sample of animal tissue comprises a receiving tube (21; 25) for receiving a sample of animal tissue,
designed to cooperate with a device for collecting comprising at least one cutting element designed (31; 34; 51) for cutting out said sample.
wherein said device for storing comprises a weighted element (22) in a first position prior to the collecting of said sample, in which said weighted element (22) is held in said device for storing,
and wherein said weighted element (22) is a sphere or a cylinder having a density greater than or equal to that of at least one specific agent (23) contained in said tube (21; 25), said weighted element being configured to take at least one second position subsequently to the collecting of said sample, in which said weighted element (22) is released in said receiving tube (21; 25),
said weighted element (22) being pushed into said tube during said collecting.

14. Method for manufacturing a device for collecting according to claim 12 or a device for storing according to claim 13,
wherein it comprises a step for inserting a weighted element (22) into said device for collecting or into said device for storing,
said weighted element (22) being a sphere or a cylinder having a density greater than or equal to that of at least one specific agent (23) contained in said tube (21; 25),
said weighted element being configured to take at least two positions, comprising a first position prior to the collecting of said sample in which said weighted element (22) is held in said device for collecting or in said device for storing and a second position subsequent to the collecting of said sample, in which said weighted element (22) is released in said tube, said weighted element (22) being pushed into said tube during said collecting.
